(19) **Europäisches Patentamt**
**European Patent Office**
**Office européen des brevets**

(11) **EP 2 859 313 B1**

(12) **EUROPEAN PATENT SPECIFICATION**

(45) Date of publication and mention
of the grant of the patent:
**07.07.2021 Bulletin 2021/27**

(21) Application number: **13796460.7**

(22) Date of filing: **30.05.2013**

(51) Int Cl.:
*G06K 9/00* (2006.01)    *G06F 17/00* (2019.01)
*G01J 1/00* (2006.01)    *G01N 30/50* (2006.01)
*G01N 30/00* (2006.01)    *G01N 27/447* (2006.01)
*G01N 21/00* (2006.01)    *G16C 20/20* (2019.01)
*G01N 21/65* (2006.01)    *G01N 21/35* (2014.01)

(86) International application number:
**PCT/US2013/043421**

(87) International publication number:
**WO 2013/181429 (05.12.2013 Gazette 2013/49)**

(54) **SYSTEM AND METHOD FOR DETERMINING THE PRESENCE OF SPECTRAL COMPONENTS IN THE SPECTRA OF MIXTURE**

SYSTEM UND VERFAHREN ZUR BESTIMMUNG DES VORHANDENSEINS SPEKTRALER KOMPONENTEN IN DEN SPEKTREN EINES GEMISCHES

SYSTÈME ET PROCÉDÉ POUR LA DÉTECTION DE LA PRÉSENCE DE COMPOSANTES SPECTRALES DANS LES SPECTRES DE MÉLANGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2012 US 201261653743 P**

(43) Date of publication of application:
**15.04.2015 Bulletin 2015/16**

(73) Proprietors:
• **Jackson, Richard**
**Nashua, New Hampshire 03062 (US)**
• **Wang, Qian**
**Nashua, New Hampshire 03062 (US)**

(72) Inventors:
• **Jackson, Richard**
**Nashua, New Hampshire 03062 (US)**
• **Wang, Qian**
**Nashua, New Hampshire 03062 (US)**

(74) Representative: **sgb europe**
**Lechnerstraße 25a**
**82067 Ebenhausen (DE)**

(56) References cited:
EP-A1- 2 366 991    EP-A2- 0 535 700
US-A- 5 023 804    US-A- 5 308 982
US-A1- 2009 210 194    US-A1- 2009 210 194
US-A1- 2011 153 226    US-A1- 2011 213 746

• **None**

**Description**

The present invention pertains to the analysis of data, and more specifically to a system and method to determine the spectral components of mixtures.

Comparing a spectrum of a sample to a reference spectrum for the verification or identification of the sample is a common practice in analytical chemistry. Examples of the types of spectra used for this purpose include but are not limited to infrared, near-infrared, Raman, mass, ultraviolet-visible, and Nuclear Magnetic Resonance (NMR) spectra. There are numerous metrics used for such spectral comparisons, including Euclidian distance, maximum distance, and correlation coefficient.

EP 2366991 A1 discloses a method for analyzing a mixture. The method includes identifying a plurality of possible components of the mixture, calculating at least one feature for at least a portion of the plurality of possible components, and calculating a probability value for at least a portion of the plurality of possible components based on the at least one feature and at least one transfer function. The method may use a partial correlation coefficient as a metric.

US 2009/210194 A1 provides a method of efficient spectral matching, in particular for multicomponent spectra. An unknown spectrum obtained from infrared or other spectroscopy can be compared to spectra in a reference library to find the best matches. The best match spectra can then each in turn be combined with the reference spectra, with the combinations also being screened for best matches versus the unknown spectrum. These resulting best matches can then also undergo the foregoing combination and comparison steps. The process can repeat in this manner until an appropriate stopping point is reached, for example, when a desired number of best matches are identified, when some predetermined number of iterations has been performed, etc.

EP 0535700 A2 discloses a method and an apparatus for comparing spectra. The disclosed method is based upon multiple linear least squares regression and is especially well-adapted for use with ICP atomic emission spectra. The multiple linear least squares regression method of determining the concentration of an analyte from a spectrum of an unknown mixture containing the analyte is improved. It is known to combine spectra of individual components of the mixture to derive a model which is then applied to the unknown spectrum. In accordance with the disclosure of this reference, first, and possibly higher derivatives of the unknown spectrum are also generated and included in the derived model.

US 2011/153226 A1 discloses methods and systems for identifying components of an unknown mixture using spectral analysis techniques. The method includes comparing the spectrum of the unknown mixture with the spectra of library compounds to obtain candidate mixture combinations. A model is generated for each of the candidate mixture combinations based on a modeling metric. A residual spectrum is computed corresponding to each of the candidate mixture combinations by removing the spectrum of each of the compounds of the candidate mixture combination from the spectrum of the unknown mixture. One or more potential compounds are identified by comparing the residual spectrum with the spectrum of library compounds.

These metrics work well when comparing the spectra of pure samples or components, or the spectra of mixtures where the sample and reference spectra contain the same components at similar concentrations. They do not perform as well however, and in fact may perform extremely poorly, when comparing the sample spectrum of a mixture to a reference spectrum of a pure component contained in that mixture, or to a reference spectrum of a mixture with the same components as in the sample spectrum, but at different concentrations. It should be noted that in this context "mixture" does not necessarily mean that the components are physically mixed, but rather that they all contribute to the spectrum.

When using the term spectrum (or its plural spectra), we refer to the data as measured and/or to any subsequent mathematical transformation of said data, to include derivatives (including first, second, third and any subsequent derivation), smoothing, baseline corrections, etc.

An object of the present invention is to provide an improved comparison method for when a sample spectrum is a mixture of components. Such a mixture may contain components that are known to be present, components that are suspected to be present but their presence needs to be confirmed, unknown components that need to be identified, or any combination thereof. The components that are known to be present may have been identified by any method, including by the application of the simple comparison metrics mentioned above, or by the application of the comparison method described herein.

The object is solved by the subject matter of the independent claim(s). The dependent claims provide preferred embodiments of the invention.

According to the invention, a computer-implemented method for determining the presence of a spectral target component in a sample spectrum (S) of a mixture of components is provided. The method comprising: performing a regression that includes a target spectrum (T) and the spectra (K') of one or more known other components, present in the sample spectrum (S) of the mixture, the target spectrum (T) being the spectrum of the target component, so that $|S = T * c_0 + K' * c' + R,$ where $c_0$ is a scalar coefficient, c' is a matrix of coefficients, and R is a residual. The method further provides: calculating an extracted spectrum E = S - K'*c', where said extracted spectrum (E) is the residual (R) in the case where $|c_0$ is set to zero, and comparing said extracted spectrum (E) to said target spectrum (T) using a comparison metric to determine the presence of the target component

Hereinafter, the terminology, embodiment" relates to the disclosure and only form part of the invention if it falls within the scope of the claims. The scope of protection is defined by the appended claims.

The present invention performs particularly well compared to other comparison metrics when the concentration of the component to be identified or confirmed is small in comparison to the concentrations of the other components. Under certain circumstances it also performs well when two or more of the components in the sample spectrum are unknown.

For the purpose of this specification, and unless otherwise noted, the term 'comprise' shall have an inclusive meaning-i.e. that it will be taken to mean an inclusion of not only the listed components it directly references, but also other non-specified components or elements. This rationale will also be used when the term 'comprised' or 'comprising' is used in relation to one or more steps in a method or process.

In one embodiment, said comparison metric is at least one of a correlation coefficient, weighted correlation coefficient, Euclidian distance or maximum distance. In yet another aspect, the weights in said weighted correlation coefficients are not all the same.

In another embodiment, the method provides comparing said extracted spectrum to the residual spectrum. In one embodiment, said method is used in an iterative fashion. In another embodiment, said regression includes a baseline spectrum or other components' spectra. In one embodiment,, said regression spectra include either derivative spectra or derivative spectra multiplied by an envelope function. In yet another embodiment, it is about using one or more regression coefficients to calculate concentrations. In one embodiment, the method further comprises at least one of the multiple spectra of a target component and the multiple spectra of a known component.

In another aspect, there are multiple targets within said mixture and different spectral regions are used for each different target. In one embodiment, a plurality of spectral regions is used. In yet another embodiment, multiple spectra of an identified or target component are factored, and a subset of the factors are included in said regression. In another embodiment, two or more regressions are performed for different target components.

In one embodiment, the method further comprising factoring multiple target spectra, projecting the spectra to be analyzed and the spectra of the known components into the factor space, performing said regression in the factor space and either comparing the extracted spectrum and the target spectra in the factor space, or projecting the extracted spectrum back to the original spectrum space and performing the comparison between the extracted spectrum and the target spectra in the original spectrum space.

In one embodiment, the method further comprises grouping a number of target spectra for each of a number of target components into clusters, performing said regression for the target spectra in each cluster, using either the mean of the target spectra, all of the target spectra, or a subset of the factors of the target spectra in each cluster, calculating the extracted spectrum for each cluster and comparing the extracted spectra to the clusters of target spectra to determine which, if any, cluster the target spectrum belongs to. In yet another embodiment either multiple spectra of a known component are included in the regressions, or a subset of factor loadings of multiple spectra of a known component is included in the regressions. In one aspect, the comparisons are performed using a correlation coefficient, a weighted correlation coefficient, a Euclidian distance, or maximum distance. In yet another embodiment it further involves comparing the extracted spectra to the residual spectra. In another embodiment, the regressions include a baseline spectrum or other component spectra. In one embodiment, a plurality of spectral regions are used. In another embodiment, the method uses one or more regression coefficients to calculate concentrations.

In one embodiment, the invention comprises factoring a number of target spectra for each of a number of target components, grouping the factors into clusters, projecting the spectra to be analyzed and the spectra of the known components into the factor space; performing said regression for the target spectra in each cluster in the factor space, using either the mean of the factors of the target spectra, or all of the factors of the target spectra, calculating the extracted spectrum for each cluster and either comparing the extracted spectra and the target spectra in the factor space, or projecting the extracted spectra back to the original spectrum space and performing the comparison between the extracted spectra and the target spectra in the original spectrum space, to determine which, if any, cluster the target spectrum belongs to.

In another embodiment, multiple spectra of a known component are included in the regression. In yet another embodiment, the comparisons are performed using a correlation coefficient, a weighted correlation coefficient, a Euclidian distance, or a maximum distance. In one embodiment, it further comprises comparing the extracted spectra to the residual spectra. In yet another embodiment, the regressions include a baseline spectrum or other component spectra. In another embodiment, a plurality of spectral regions is used. In another embodiment, the method comprises using one or more regression coefficients to calculate concentrations.

In another aspect, a non-transitory computer-readable medium for storing computer code for a method for determining the presence of spectral components in a mixture is provided, said method comprising performing a regression that includes the target spectrum and one or more known other component spectra present in a mixture, calculating the extracted spectrum, where said extracted spectrum is the residual where the coefficient for the target spectrum is set to zero, and comparing said extracted spectrum to said target spectrum using a comparison metric.

Other features and advantages of the present invention will become apparent upon examining the following detailed description of an embodiment thereof, taken in conjunction with the attached drawings.

**FIG. 1** shows an illustration of the spectrum of 100 ppm Nitric Oxide (NO) in the presence of water vapor (dashed line) and the spectrum of water vapor (solid line), according to an exemplary embodiment of the invention.

**FIG. 2** shows an illustration of the spectrum of 100 ppm NO in the presence of water vapor (solid line) and the residual spectrum (dashed line), according to an exemplary embodiment of the invention.

**FIG. 3** shows an illustration of the target spectrum of NO (dashed line) and extracted spectrum (solid line), according to an exemplary embodiment of the invention.

**FIG. 4** shows an illustration of the residual spectra used to calculate the partial correlation coefficient for NO.

**FIG. 5A** - **5C** show an illustration of the spectra from the analysis of carbon monoxide and nitrous oxide in the 100 ppm NO sample spectrum. **FIG. 5A** Target spectrum of nitrous oxide (dashed line) and extracted spectrum from second analysis (solid line). **FIG. 5B** Target spectrum of carbon monoxide (dashed line) and extracted spectrum from first analysis (solid line). **FIG. 5C** Sample spectrum. All above according to exemplary embodiments of the invention.

**FIG. 6** shows an illustration of the spectra acquired using a Raman spectrometer, of an aqueous solution of a drug within a bottle (solid line), that of the bottle (dashed line), the water (dash-dot line), and the target spectrum of the drug (dotted line), according to an exemplary embodiment of the invention.

**FIG. 7A - 7C** show an illustration of the target (**7A**), extracted (**7B**) and residual (**7C**)) spectra from the analysis of the Raman spectrum of an aqueous solution of a drug in a bottle. All above according to exemplary embodiments of the invention.

**FIG. 8** shows an illustration of the target spectrum of the drug (top) and the extracted spectrum from untransformed data (bottom), according to an exemplary embodiment of the invention.

**FIGS. 9 - 10** shows example clusters for spectra of mixtures **FIG. 9** and spectra of target components **FIG. 10.**

The above-described and other features will be appreciated and understood by those skilled in the art from the following detailed description, drawings, and appended claims.

In one embodiment, let S be the spectrum of a sample that consists of n components that have spectra $K_1...K_n$. Assuming the Beer-Lambert law is obeyed, the sample spectrum can be modeled as;

$$S = \sum_{i=1}^{n} \left( c_i \bullet K_i \right) + R$$

Where K is the matrix of reference spectra of the sample components, $c_1...c_n$ are unknown coefficients and R is a residual, or error, term. The least squares solution to this equation for the coefficients $c_1...c_n$ can be found by standard matrix algebra. This is a well-known technique in the field, and in standard chemometrics terminology is usually referred to as a Classical Least Squares (CLS), or K-matrix, regression. It will be appreciated that while least squares is the most common method used to determine the coefficients $c_1...c_n$, other techniques may be employed. For example, maximum likelihood could be used to achieve a similar result. In one embodiment, in the equation above it is assumed that the data points for all spectra have common abscissa values. If this is not the case then the spectra can be interpolated to common abscissa values prior to the regression. Constraints, such as non-negativity, may be applied in the regression.

As is well known in the art a baseline can be added to the regression by adding one or more columns to the matrix K. For example, adding a column of ones (1 or 1's) will include an offset in the regression; adding a column of ls and a column containing the abscissa values, such as the wavenumber (or wavelength) values, will include a linear baseline in the regression. Adding a column of Is, a column containing the wavenumber (or wavelength) values and a column containing the square of the wavenumber (or wavelength) values will include a quadratic baseline in the regression, etc.

These additional columns can be considered to be additional reference spectra, with a corresponding increase in n. Other reference spectra can also be added to the matrix K to account for measurement errors or other variance in the spectral data that would otherwise not be accounted for in the model. For example, Ivaldi et al US 5,308,982 describes the inclusion of derivative spectra, or derivative spectra multiplied by envelope functions, to account for changes in the abscissa between the sample spectrum S and the spectra in K.

In the context of this application, the inclusion of a derivative of the sample spectrum in the regression will compensate for shifts in the abscissa between the sample spectrum S and the spectra in K. The simple first derivative spectrum can be replaced by the first derivative spectrum multiplied by a straight line envelope function which varies from - I at the left to +I at the right. The inclusion of this modified derivative in the model will allow a scale expansion/compression with zero mean shift to be compensated. Clearly, the simple shift compensation and the expansion/ compression compensation can be combined to compensate for a more general change of scale. The effects of any complex distortion of the abscissa scale may be modeled by including higher orders of envelope function. A constant envelope function corresponds to a simple shift. A linear envelope function corresponds to a scale compression or expansion. A parabolic envelope function corresponds to compression in one half of the spectrum and expansion in the other half, and so on. In fact, the entire family of compensating functions consists of all the orders of derivative spectrum multiplied each in combination with one of the orders of envelope function. Knowledge of likely causes of spectrum distortion will aid in the restriction of this set to manageable proportions.

If each spectrum contains m data points then we can write this in matrix notation as;

$$S = K \cdot c + R$$

Where S and R are $m \times 1$ matrices, K is a $m \times n$ matrix of reference spectra, and c is an $n \times 1$ matrix of coefficients. Often, all of the components represented in K are known to be present, and the objective of the regression is to find the coefficients c, that can then be used to calculate their relative concentrations. In certain cases, however, one of the components may be an unknown component that needs to be identified, or a suspected component whose presence in the mixture needs to be confirmed. If we designate this component as the target component, and the spectrum of this component as T, the target spectrum, then for convenience we can rewrite the equation as;

$$S = T \cdot c_0 + K' \cdot c' + R$$

Where S, T and R are $m \times 1$ matrices, K' is an $m \times (n-1)$ matrix of reference spectra of known components that does not contain the spectrum in T, c' is an $(n-1) \times 1$ matrix of coefficients, and co is a scalar coefficient. It should be noted that in the regression there is mathematically no distinction between the spectra of the target and known components. The distinction is made here only for the purposes of clarity in describing the method.

It is common practice to judge the quality of the model represented by the above equations by examining the size of the residuals, R. If the residuals are small relative to the spectrum S then the model is deemed to be good. Here, "small" may mean small in an average, or root mean square (RMS), sense, or it may mean that the residuals must be small at all points in the spectrum (See for example Ivaldi et al US 5,308,982).

Other methods may also be used to judge the quality of the model, for example calculating a comparison metric such as correlation coefficient between the spectrum S and the sum spectrum $T \cdot c_0 + K' \cdot c'$ (as seen in Ritter et al US 7,698,098). As long as the term $T \cdot c_0$ is greater than or substantially similar in magnitude to the term $K' \cdot c'$ (i.e. the target spectrum, T, is a substantial fraction of the sample spectrum) the comparison metrics given above are often, although not always, reasonable indicators of the presence of the target component.

However, if the contribution of the target component to the spectrum S is very small, the comparison metrics given above are very poor indicators of the presence of the target component. This is because the regression of only the spectra of the known components, K', will result in a very good fit to the sample spectrum S, resulting in a very small residual (close to zero) and a high correlation coefficient (close to one). Addition of a spectrum of a target component to this regression is certain to reduce the magnitude of the residuals and increase the correlation coefficient, because the number of coefficients in the regression has been increased, but the reduction in the magnitude of the residuals and the increase in the correlation coefficient will be small because the residuals cannot be less than zero and the correlation coefficient cannot be greater than one. This makes it very difficult to judge whether the improvement in the fit is significant, and therefore indicates the presence of a target component.

Another metric that has been used to indicate whether or not a component is present in a mixture is a partial correlation coefficient (see Appl. Spec., 66, p334, 2012, as well as Botonjic-Sehic et al US Pat. Appl. 2011/0213746). The partial correlation coefficient between the sample spectrum and the target spectrum is the correlation coefficient between the residual when the sample spectrum is regressed against the spectra of all components other than the target spectrum (i.e. when S is regressed against K'), and the residual when the target spectrum is regressed against the spectra of all other components (i.e. when T is regressed against K').

However, the partial correlation coefficient is not as flexible as the method described herein. The only appropriate comparison between the two residuals is correlation, because the residuals may be on very different scales and have large baseline differences. The two residuals are also not suitable for visual comparison, because both differ from the known quantity, i.e. the reference spectrum of the target component, in unpredictable ways. Visual interpretation of the residuals by an analyst, even one that is a skilled spectroscopist, is therefore difficult or impossible.

The approach described herein is to first calculate an approximation to the target spectrum by first performing a regression that includes the target and known spectra ($S = T c_0 + K' c' + R$), and then calculating a residual with the coefficient for the target spectrum, $c_0$, set to 0, defined as the extracted spectrum, or E;

$$E = S - K' \cdot c'$$

This can be compared to the expression for the residual, R;

$$R = S - T \cdot c_0 - K' \cdot c'$$

The residual, R, will be small if either the target component is not present and $K' \cdot c'$ is a good approximation to S (in which case the coefficient $c_0$ will be close to zero), or if the target component is present and $T \cdot c_0 + K' \cdot c'$ is a good approximation to S. As noted above, this is therefore not a good indicator of the presence of the target component. The extracted spectrum, E, will also be small, and will resemble R, if the target component is not present and $K' \cdot c'$ is a good approximation to S (i.e. the spectra of all components present in the mixture are included in K'). However, if the target component is present in the sample at any significant concentration and $T \cdot c_0 + K' \cdot c'$ is a good approximation to S then the extracted spectrum will resemble the spectrum of the target component. Also, if the target component is not present and $K' \cdot c'$ is not a good approximation to S because another component is present that was not included in the regression, the extracted spectrum will not resemble either R or the target spectrum.

Comparison of the extracted spectrum to the target spectrum, typically scaled by the regression coefficient $c_0$, can therefore be used as a reliable indicator of the presence of the target component. The comparison could be visual, by overlaying the two spectra on the computer screen, or it could be a calculated metric such as Pearson's correlation coefficient, a weighted correlation coefficient, the Euclidean distance, or the maximum absolute difference between the spectra.

In one embodiment the comparison metric is a weighted correlation coefficient, where the weights are used to emphasize certain features or aspects of the data, whilst suppressing others. For example, the weights could be set equal to the absolute value of the target spectrum, thereby emphasizing spectral regions where the target spectrum has features and suppressing regions where it does not. There are many other possible ways to calculate the weights, depending on which features or aspects of the data are to be emphasized or suppressed, with the choice of weighting scheme depending on the nature of the problem that the method is being applied to. Also, it may sometimes be useful to make a similar comparison between the extracted spectrum and the conventional residual, R.

As long as the spectra of all components present are included in the regression, the method described above will also work if the sample contains more than one suspected component that needs to be confirmed In this case one of the target spectra is T, all the other target spectra are included in K', the extracted spectrum is calculated and compared to the target spectrum. This is then repeated for each of the other target spectra. If some or all of the suspected components are present then the extracted spectra for the components that are present will resemble the corresponding target spectra, and the extracted spectra for the components that are not present will resemble the residual, R.

The method described above may also work with more than one unknown component, especially if the principle spectral features of the unknown components are in different spectral regions. In this fashion, the individual components in a mixture may be identified.

An additional advantage of this method is that the coefficients from the regression may be used to calculate estimated concentrations, or relative concentrations, of the components.

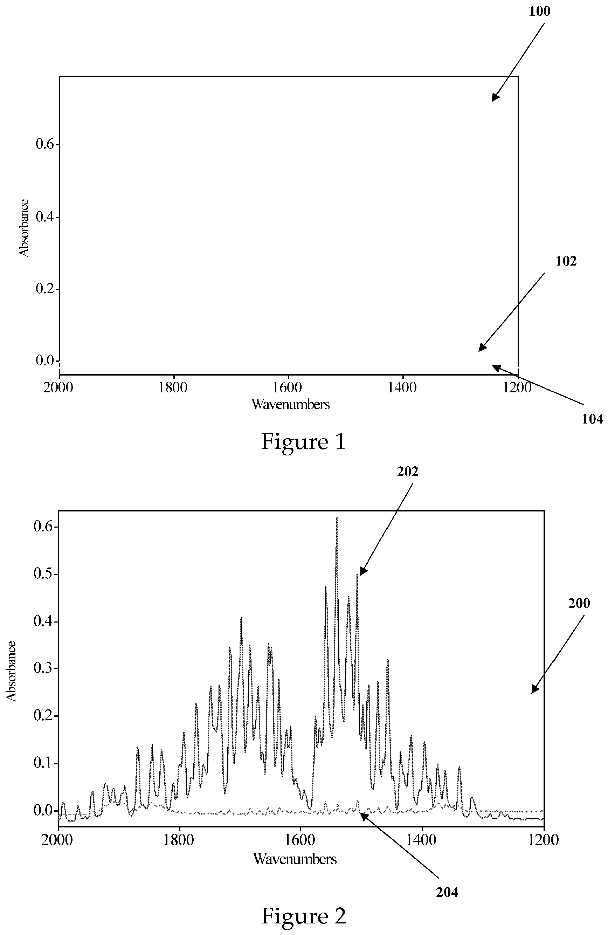

As an example, we will consider the infrared spectrum of a low concentration of nitric oxide (NO) in the presence of a high concentration of water vapor. FIG. 1 shows 100 a sample spectrum of 100 ppm NO in the presence of water vapor 102 (dashed line) and a spectrum of pure water vapor 104 (solid line), over the region 1200-2000 $cm^{-1}$. Since the spectrum of water vapor varies with temperature, the two spectra were collected using the same instrument on the same day.

If the water vapor spectrum plus a linear baseline is regressed against the sample spectrum over this region the RMS residual is 0.006668 and the correlation coefficient between the fitted (i.e. sum) spectrum and the sample spectrum is 0.998229. **FIG. 2** shows **200** the sample spectrum **202** and the residual R **204,** on the same scale. The residual **204** does show features that do not resemble random noise, and a skilled spectroscopist may interpret some or all of these as missing components that should be included in the regression. However, in many real applications of the method the analyst conducting the measurement will not be a skilled spectroscopist, in which case such a visual inspection will not be a practical indicator of a component that is missing from the regression.

The above visual inspection is also not useful if the objective is an automated response that depends on the results of the analysis, because then a numeric metric is required.

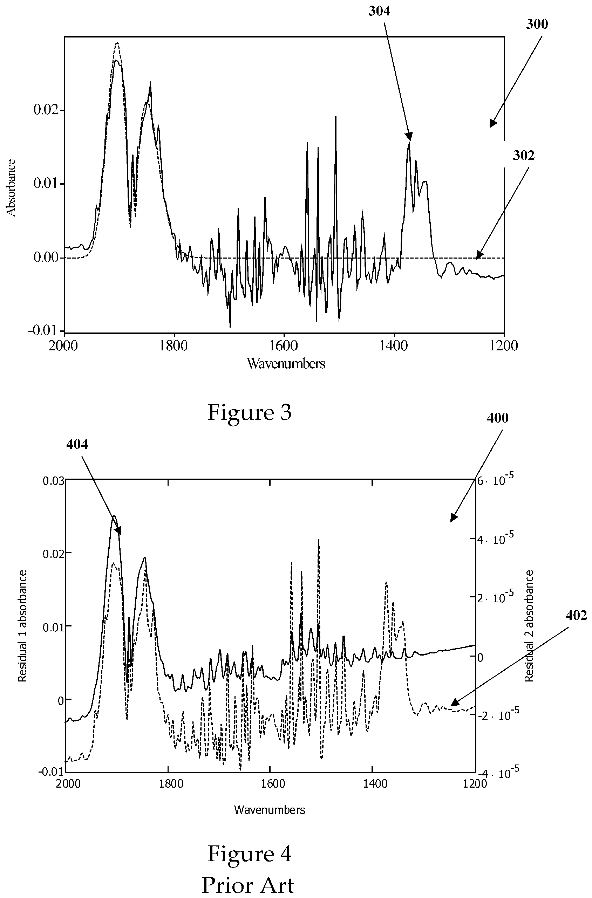

If a reference spectrum of NO is also included in the regression, the RMS residual decreases to 0.003881, and the correlation coefficient between the fitted spectrum and the sample spectrum increases to 0.999401. However, changes of this magnitude in these metrics are not sufficient to indicate whether or not NO is present, because adding a spectrum to the regression is guaranteed to decrease the RMS residual and increase the correlation coefficient. **FIG. 3** shows **300** the target (or reference) spectrum of NO scaled by the regression coefficient **302** (dashed line) and the extracted spectrum from the regression **304** (solid line). The correlation coefficient between these spectra is 0.860623, which is high enough to give a strong indication that NO is present. This is clearly confirmed by a visual comparison, which also shows why the correlation coefficient is not even higher. The extracted spectrum shows features between 1400 and 1800 cm$^{-1}$ from uncompensated water vapor absorption, and a feature between 1400 and 1800 cm$^{-1}$ that is due to trace quantities of sulfur dioxide ($SO_2$) that are in fact known to be present in this sample. The features from the water vapor occur because the water vapor is more than one order of magnitude more strongly absorbing than the NO, and therefore even a small difference between the reference spectrum and the sample spectrum (as for example could be caused by temperature changes) will lead to a residual that is comparable in magnitude to the NO absorption.

For comparison purposes, the partial correlation between the NO target spectrum and the 100ppm NO sample spectrum is 0.812922. This is not as high as the correlation between the target spectrum and the extracted spectrum, but does also give an indication that NO is present in the sample. **FIG. 4** shows **400** the two residual spectra used to calculate the partial correlation coefficient for the NO target spectrum and the 100ppm NO sample spectrum. A first residual **402** (dashed line) is obtained by regressing the sample spectrum against the water vapor spectrum and a linear baseline. A second residual **404** (solid line) is obtained by regressing the NO target spectrum against the water vapor spectrum and a linear baseline.

In contrast to the extracted and target spectra, interpretation of these first and second residuals is difficult because they both contain spectral features that are not associated with NO, and without comparison to the reference spectrum of NO it is not possible to know which features these are. Furthermore, because of the very different scales of the two residuals (about two orders of magnitude) many potential comparison metrics, such as maximum difference, are not applicable. In this example the partial correlation coefficient and the associated residuals are therefore clearly not as reliable an indicator of the presence of NO as the comparison (i.e. calculated correlation coefficient and/or visual comparison) of the extracted spectrum to the target spectrum.

Using a smaller spectral range, or more than one spectral range, for the comparison would clearly reduce the problem of uncompensated features and trace components that are not included in the regression reducing the correlation between the extracted and target spectra. If the component is a suspected component and the objective is to confirm its presence then choosing a smaller, more suitable, spectral range based on characteristic features in the target spectrum will typically be easy.

However, if the component is an unknown component, and the objective is to identify it, choosing too small a spectral range may mean that some of the target spectra have no characteristic absorption features in that spectral range. A variation of the method described above is therefore to use different spectral ranges for different target components. There are many possible methodologies or algorithms to choose suitable spectral ranges for each target component, and each target component could have one, two, or more, spectral ranges associated with it. Because certain comparison metrics may depend on the size of the total spectral range it may then desirable, although not necessary, to have the total spectral range for all target components be the same size.

As an example we will consider the same sample spectrum as used above, i.e. 100 ppm NO in water vapor, but rather than comparing to only one target spectrum (that of NO), it will be compared to a database containing over 400 target spectra. In this example the sample spectrum was analyzed over a wider spectral range than above, 1000 to 2400 cm$^{-1}$, and spectra of both water vapor and carbon dioxide were included as known components. A series of regressions was then performed, each regression including the spectrum of the target component, the spectra of the two known components, and a linear baseline. The spectral range for each target component was set as $\pm75$ cm$^{-1}$ of the strongest absorption within the range 1000 to 2400 cm$^{-1}$. The top ten matches, as defined by the correlation coefficient between the extracted and target spectra, are shown below in Table 1.

Table 1. Top ten matches for 100 ppm NO in water vapor.

| | Target Name | Approximate Concentration (ppm) | R1 | R2 | R3 | RMS Residual |
|---|---|---|---|---|---|---|
| 1 | Nitric oxide | 96.2 | 0.994330 | 0.106340 | 0.999717 | 0.000890 |
| 2 | Carbon monoxide | 44.0 | 0.992521 | 0.122076 | 0.989108 | 0.000961 |
| 3 | Sulfur dioxide | 5.1 | 0.983485 | 0.180988 | 0.999733 | 0.000990 |
| 4 | Dimethylamine | 2.0 | 0.811840 | 0.583879 | 0.929256 | 0.000265 |
| 5 | Ethyl chloroformate | 0.1 | 0.810476 | 0.585772 | 0.943240 | 0.000263 |
| 6 | Methyl chloride | 101.9 | 0.753454 | 0.657501 | 0.996764 | 0.003527 |
| 7 | Methyl methacrylate | 0.2 | 0.753233 | 0.657754 | 0.936591 | 0.000287 |
| 8 | Allyl alcohol | 0.3 | 0.740650 | 0.671891 | 0.949891 | 0.000102 |
| 9 | Acetylene | 57.7 | 0.728916 | 0.684603 | 0.995680 | 0.004059 |
| 10 | Diisopropyl ether | 0.3 | 0.714094 | 0.700050 | 0.918241 | 0.000248 |

R1 is the correlation coefficient between the extracted spectrum and the target spectrum, R2 is the correlation coefficient between the residual and the extracted spectrum, and R3 is the correlation coefficient between the sample spectrum and the fitted (sum) spectrum. For the top three matches R1 is greater than 0.98, but for the fourth match it drops to only 0.81. Conversely, for the top three matches R2 is less than 0.2, but for the fourth match increases to almost 0.6. The top three matches are in fact known to be present in the sample, whereas the other matches are known not to be present. R1 and R2 are therefore good indicators of the presence of a target component in the sample.

The correlation coefficient between the sample spectrum and the fitted spectrum, and the RMS residual for the regression, are also shown in the table. It can be seen that there are target components that are known not to be present for which this correlation coefficient is higher than is it for components that are known to be present. Similarly, there are target components that are known not to be present for which the RMS residual is lower than is it for components that are known to be present. As has already been stated, these two metrics are therefore very poor indicators of the presence of a target component. The estimated concentrations in the table are calculated based on the regression coefficients. The estimated concentration for NO is in good agreement with the known concentration of 100 ppm.

A further variation of the method is to apply it in an iterative fashion. Once certain components have been established to be present in the sample spectrum based on R1 and R2, their spectra can be added to the list of known components. The sample spectrum can then be reanalyzed to look for lower concentration unknown components that may not have been found in the previous analysis, because the regression may not have included all components that are actually present in the sample spectrum. This procedure can be repeated until no further components are identified. As an example, the spectra of nitric oxide, carbon monoxide, and sulfur dioxide were added to the list of known components and removed from the list of target spectra used in the first analysis, and a new series of regressions was performed. Table 2 shows the top ten matches from the second analysis of the 100 ppm NO sample spectrum.

Table 2. Top ten matches for second analysis of 100 ppm NO in water vapor.

| | Target Name | Approximate Concentration (ppm) | R1 | R2 | R3 | RMS Residual |
|---|---|---|---|---|---|---|
| 1 | Nitrous oxide | 1.8 | 0.996193 | 0.087175 | 0.999991 | 0.000290 |
| 2 | Acetylene | 16.5 | 0.845722 | 0.533623 | 0.999841 | 0.000780 |
| 3 | Allyl alcohol | 0.3 | 0.801722 | 0.597697 | 0.955974 | 0.000096 |
| 4 | Propargyl alcohol | 0.2 | 0.750171 | 0.661243 | 0.954137 | 0.000096 |
| 5 | Dichlorosilane | 1.9 | 0.702996 | 0.711193 | 0.999679 | 0.001538 |
| 6 | 1,2-Dichloro-1-fluoroethane | 0.3 | 0.697152 | 0.716923 | 0.954861 | 0.000094 |
| 7 | Dimethyl carbonate | 0.1 | 0.685309 | 0.728252 | 0.999769 | 0.000555 |

(continued)

|  | Target Name | Approximate Concentration (ppm) | R1 | R2 | R3 | RMS Residual |
|---|---|---|---|---|---|---|
| 8 | Nitrogen dioxide | 1.4 | 0.673335 | 0.739337 | 0.999612 | 0.003023 |
| 9 | Phosgene | 0.5 | 0.660197 | 0.751092 | 0.999797 | 0.001004 |
| 10 | Methyl alcohol | 0.2 | 0.656569 | 0.754266 | 0.952319 | 0.000098 |

The values of R1 and R2 clearly indicate that nitrous oxide ($N_2O$) is also present in the sample, but at a very low concentration of less than 2 ppm. This component is in fact also known to be present in trace quantities. The presence of this component was masked in the first analysis because the main absorption band of $N_2O$ overlaps the main absorption band of carbon monoxide, which is present at much higher concentrations. The regression for $N_2O$ in the first analysis therefore did not include the spectra of all the components with significant absorption in the spectral range used for the analysis.

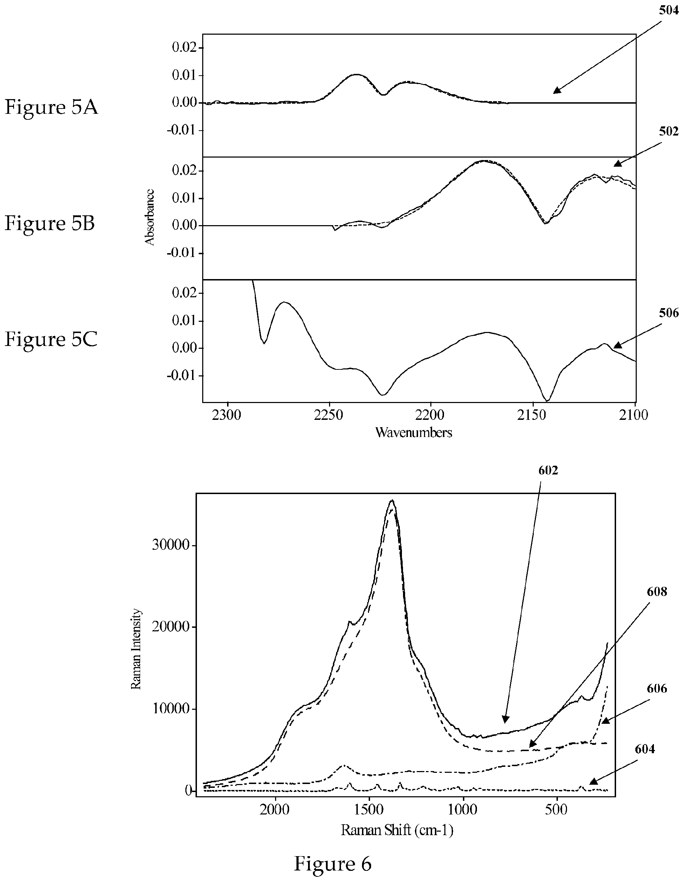

With both nitrous oxide and carbon monoxide included in the regression a revised concentration of 41.2 ppm can also be calculated for carbon monoxide (the concentration of the other components identified in the first analysis are unchanged). The sample spectrum **506,** as well as the target and extracted spectra (dashed and solid lines, respectively) for carbon monoxide and nitrous oxide are shown in **FIGS. 5A - 5C.** The spectra for carbon monoxide **502** are from the first analysis, the spectra for nitrous oxide **504** are from the second analysis. Features in the sample spectrum that are not due to either carbon monoxide or nitrous oxide are due to water vapor and carbon dioxide. Visual inspection clearly confirms the presence of both components.

It is possible that in some cases the spectrum of the target component, or one or more spectra of the known components, or both, may exhibit significant variation from one measurement to the next. This may result in a large residual from the regression, which will mask the presence of the target component even if it is present, or lead to errors in its estimated concentration. There is more than one variation of the comparison method that will minimize or eliminate these effects.

In some cases it may be possible to minimize or even eliminate the effects of variation in the spectra by preprocessing or transforming the spectra prior to applying the comparison method. For example, the sample spectrum may include non-reproducible fluctuations that are much broader that the spectral features of the target spectrum, but are nevertheless too complex to be modeled by the baselines described above. In this case calculating the derivatives of all the spectra that are to be included in the regression will result in a more reliable indication of the presence of the target component, and more accurate estimates of the relative proportion of the components. This is because the derivative is a measure of the slope, and therefore emphasizes rapidly varying features relative to slowly varying features. The extracted spectrum, as defined above, is then clearly also a derivative, but it is possible to use the regression coefficients to calculate an extracted spectrum that is not a derivative.

Such an extracted spectrum can be calculated by using the preprocessed or transformed spectra to calculate the regression coefficients in $S = T \cdot c_0 + K' \cdot c' + R$, and then using these coefficients with the original spectra to calculate the extracted spectrum using $E = S - K' \cdot c'$. The advantage of calculating an extracted spectrum using the untransformed data is that it is often easier for an analyst to visually compare extracted and target spectra that have not been transformed, even though a mathematical comparison may indicate a poor match (e.g. a low correlation coefficient). Many other preprocessing steps or transformations, for example smoothing, are also possible and known in the art.

An example that shows the advantage of preprocessing the data before applying the comparison method is the Raman spectrum of a dilute (0.5%) aqueous solution of an injectable drug, acquired through the wall of a glass bottle. Because the bottle is one of the components that contribute to the sample spectrum the comparison method requires that a spectrum of the bottle alone be included in the regression. The spectrum of another empty bottle can be easily acquired, but the glass exhibits photoluminescence that is not completely reproducible between different bottles. This photoluminescence is very intense compared to the Raman peaks from the drug, but it also has much broader features. The sample spectrum of the drug in the bottle **602** (solid line) and the spectra of the three components, drug **604** (dotted line), water **606** (dash-dot line), and the bottle **608** (dashed line), are shown in **FIG. 6.**

If the target spectrum is the spectrum of the drug **604,** the known spectra are the spectra of water **606** and the bottle **608,** and a curved baseline is included in the regression, then the correlation coefficient between the extracted spectrum and the target spectrum is 0.5631, whereas the correlation coefficient between the extracted spectrum and the residual is 0.8261, implying that the drug spectrum is not a component of the sample spectrum.

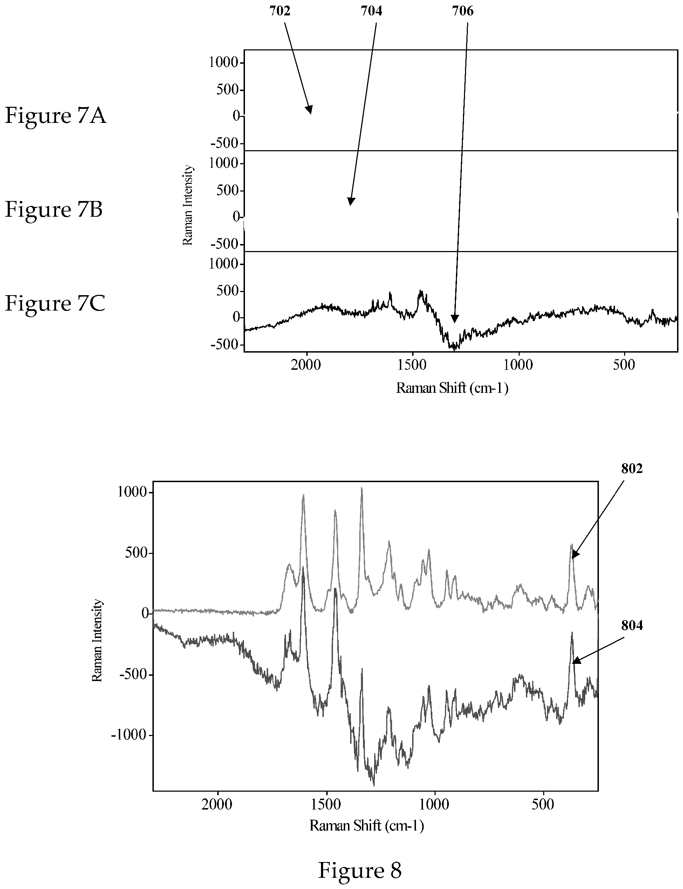

However, as shown in **FIG. 7,** both the extracted spectrum **704** and the residual spectrum **706** are dominated by the difference between the photoluminescence of the empty bottle and the bottle containing the drug solution, which leads to broad spectral features in both spectra that are not present in the target spectrum **702.** If the spectra are first preprocessed by taking their derivatives, and the curved baseline is removed from the regression, then the correlation coefficient between the extracted spectrum and the target spectrum is 0.9335 and the correlation coefficient between the extracted spectrum and the residual is 0.3588, indicating that the drug is in fact present in the solution. **FIG. 8** shows the target spectrum **802** and the extracted spectrum **804** from the untransformed data. Despite the large baseline variations, visual inspection clearly confirms the presence of the drug.

It is possible to further improve the comparison above by using a weighted correlation coefficient. The extracted spectrum has significant noise, which reduces the correlation coefficient between the extracted spectrum and the target spectrum. However, because the target spectrum has peaks of varying intensity some spectral regions are more useful for determining the presence of the target component than others. We can therefore calculate a weighted correlation coefficient that puts more emphasis on regions with greater spectral intensity. There are many ways to weight the correlation coefficient, for example the weighting schemes described in "Self-Weighted Correlation Coefficients and Their Application to Measure Spectral Similarity", Appl. Spec., Vol 63, pp 916-919 (2009), but for this example we shall use weights that are simply proportional to the square of the intensity of the target spectrum. When we use such a weighted correlation coefficient for the comparison, the correlation coefficient between the extracted spectrum and the target spectrum is 0.9754 and the correlation coefficient between the extracted spectrum and the residual is 0.1718, giving a very clear indication that the drug is present in the solution.

Another variation of the method that may minimize or eliminate the effects of spectral variation between measurements is to include multiple spectra of the target component or a known component, or both, in the regression. If multiple spectra of a known component are included in the regression then we simply add more columns to K' and more rows to c' in the equations $S = T \cdot c_0 + K' \cdot c' + R$, $R = S - T \cdot c_0 - K' \cdot c'$ and $E = S - K' \cdot c'$. If multiple spectra of a target component are included in the regression then for p spectra of a target component we can rewrite the regression equation as;

$$S = T' \cdot c_t + K'' \cdot c'' + R$$

Where S, and R are $m \times 1$ matrices, T' is an $m \times p$ matrix of spectra of the target component, K" is an $m \times (n-p)$ matrix of known spectral components that does not contain the spectra in T', c" is an $(n-p) \times 1$ matrix of coefficients, and $c_t$ is a $p \times 1$ matrix of coefficients. The extracted spectrum is calculated by simultaneously setting all the coefficients for the spectra of the target component, $c_t$, to 0. The equations for the extracted spectrum and residual become;

$$E = S - K'' c''$$

$$R = S - T' \cdot c_t - K'' \cdot c''$$

There is no longer a single spectrum of the target component, but $T' \cdot c_t$ is a weighted average of the spectra of the target component that best matches the extracted spectrum. The correlation coefficient, or other comparison metric, can therefore be calculated between this weighted average target spectrum and the extracted spectrum. If multiple spectra of either a target component or a known component are included in the regression then the concentration, or relative concentration, of that component can be calculated using a suitably weighted sum of their regression coefficients.

If a large number of target spectra or spectra of one or more known components are available, then it may be advantageous to first factor these spectra using one of several methods, including Principle Component Analysis (PCA). Factoring the spectra projects them into a new space in which it is often possible to choose a subset of the factors such that noise is reduced but only a small amount of other information is lost. The factor loadings for the subset of factors, rather than the original spectra, are then used in the equations $S = T \cdot c_0 + K' \cdot c' + R$, $R = S - T \cdot c_0 - K' \cdot c'$ and $E = S - K' \cdot c'$; or the equations $S = T' \cdot c_t + K'' \cdot c'' + R$, $R = S - T' \cdot c_t - K'' \cdot c''$ and $E = S - K'' \cdot c''$.

The method described can also be incorporated into more sophisticated chemometics algorithms that are well known, including but not limited to Discriminant analysis and SIMCA. If the spectrum of an unknown sample is to be compared to reference spectra, it is well known that the reference spectra will always exhibit some variation between measurements and reference samples. If there are several examples of each reference spectrum available then algorithms exist that can group the reference spectra into clusters. An unknown sample is then compared to these clusters to determine which, if any, it is likely to belong to. Many of these algorithms rely on factoring the data to first reduce the dimensionality. The data is often factored into its principal components, although other factoring schemes are also possible. The factoring may also be performed on all the reference spectra at once, or each group of reference spectra may be factored independently.

When using these algorithms, if the unknown spectrum is the spectrum of a mixture of components then normally the reference spectra must also be spectra of mixtures of components, even if not all of the components in the mixture are of interest. Further, if a component that is of interest, i.e. a target component, is measured in the presence of other components, and the spectra of one or more of the other components exhibit significant variation, then to account for that variation a large number of reference spectra must be measured. This is required even if the spectrum of the target component itself exhibits very little variation.

It is also possible that the target component in each reference sample will be different, but the other components will be the same, in which case if the spectra of one or more of the other components exhibit significant variation then a large number of spectra will be required for every reference sample, resulting in a very large total number of spectra that must be measured. Again, this will be required even if the spectra of the target components exhibit little variation.

The method described can be used to improve these chemometric algorithms. Rather than measuring spectra of the reference mixtures, spectra of the individual components of the reference mixtures are measured instead. This will often be possible even if it is not possible, or practical, to measure the spectra of the individual components of the unknown mixture. If the target components exhibit little variation then fewer spectra of each will be required. A large number of spectra will be required for any other component that does exhibit significant variation, but if these components are common to all the reference mixtures then the total number of spectra required may be significantly reduced.

If the spectra of the mixtures exhibit more variation than the spectra of the target components, then the clusters representing the mixture spectra will be larger than the clusters representing the target spectra. Further, if the components in the mixtures other than the target component are common to all of the mixtures, then the spectra of the mixtures will be more similar than the spectra of the target components. The clusters of the mixture spectra will therefore be larger and closer together than the spectra of the target components, and more likely to overlap.

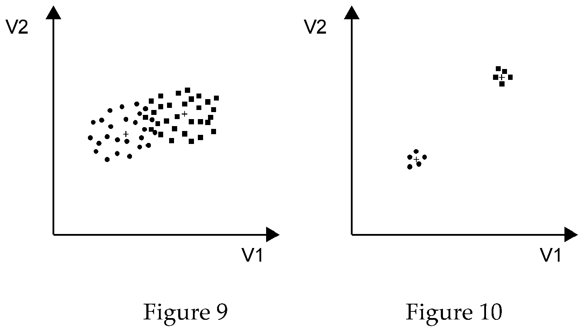

**FIGS. 9 - 10** show example clusters. For simplicity clusters for only two mixtures or target components are shown, and the clusters are defined using only two variables. However, there can be any number of clusters, any number of variables can be used to define the clusters, and there can be many different algorithms that can be used to group the spectra into clusters. The variables may be spectral intensities at different wavelengths (or wavenumbers), or if the spectra were factored they may be factor scores. Each spectrum of a mixture or a target component is represented as a point, with the different mixtures or target components being distinguished as circles or squares.

If the chemometric algorithm does not factor the data then a regression can be performed and extracted and residual spectra calculated for each target component using the appropriate equations $S = T' \cdot c_t + K'' \cdot c'' + R$, $R = S - T' \cdot c_t - K'' \cdot c''$ and $E = S - K'' \cdot c''$. The spectrum of the target component used in the regression and calculation of the extracted spectrum could be the mean of all the spectra of the target component, or all of the spectra of the target component could be included in the regression, or, as described above, a subset of factor loadings of the spectra of the target component could be included in the regression.

Also, either all of the spectra of a known component could be included in the regression, or a subset of factor loadings of the spectra of the known component could be included in the regression. The extracted spectrum for each target is compared to the corresponding cluster of target spectra to determine which, if any, the target component in the sample belongs to. Note that the algorithm used to group the spectra into clusters and the method used to compare the extracted spectra to the target spectra can be the same as in the original chemometric algorithm. The only difference is that instead of comparing the unknown mixture spectrum to the clusters of reference mixture spectra, the extracted spectra are compared to the clusters of target spectra. The extracted spectra could also be compared to the target and residual spectra using one of the comparison metrics described above.

If the chemometric algorithm factors the target spectra then the spectrum of the unknown sample and the spectra of the known components can be projected into the same factor space as the target spectra. Equations $S = T' \cdot c_t + K'' \cdot c'' + R$, $R = S - T' \cdot c_t - K'' \cdot c''$ and $E = S - K'' \cdot c''$ can then be used to perform the regression and calculate the residual and extracted spectra in the factor space in a way exactly analogous to the way they would be used with spectra that had not been factored. The comparison between the extracted spectra and the clusters of target spectra can then be performed using the same method as in the original chemometric algorithm. The extracted and residual spectra could also first be projected back to the original spectrum space, and the extracted spectra could then be compared to the target spectra and the residual spectra using one of the comparison metrics described above.

Incorporating the extracted spectrum method into these chemometric algorithms has clear advantages. In addition to potentially significantly reducing the number of reference spectra that must be acquired, the clusters are less likely to overlap with each other and a correct identification is therefore more likely.

The method and/or system described above can also be embodied in the form of computer program code containing instructions embodied in tangible media, such as floppy diskettes, CD-ROMs, hard drives (whether local or in the cloud), or any other computer-readable storage medium, wherein, when the computer program code is loaded into and executed by a computer, the computer becomes an apparatus capable of executing the method and/or system.

The present method and/or system can also be embodied in the form of computer program code, for example, whether stored in a storage medium, loaded into and/or executed by a computer, or as data signal transmitted whether a modulated carrier wave or not, over some transmission medium, such as over electrical wiring or cabling, through fiber optics, or via electromagnetic radiation, wherein, when the computer program code is loaded into and executed by a computer, the computer becomes an apparatus capable of executing the method and/or system . When implemented on a general-purpose microprocessor, the computer program code segments configure the microprocessor to create specific logic circuits.

It should be emphasized that the above-described embodiments of the present invention, particularly any "preferred embodiments" are merely possible examples of the implementations.

The scope of the present invention is defined by the appended claims.

## Claims

1. A computer-implemented method for determining the presence of a spectral target component in a sample spectrum (S) of a mixture of components, said method comprising:

   performing a regression that includes a target spectrum (T) and the spectra (K') of one or more known other components, present in the sample spectrum (S) of the mixture, the target spectrum (T) being the spectrum of the target component, so that
   $S = T * c_0 + K' * c' + R$, where $c_0$ is a scalar coefficient, c' is a matrix of coefficients, and R is a residual
   calculating an extracted spectrum $E = S - K'*c'$,
   where said extracted spectrum (E) is the residual in the case where $c_0$ is set to zero; and
   comparing said extracted spectrum (E) to said target spectrum (T) using a comparison metric to determine the presence of the target component.

2. The method of claim 1, wherein said comparison metric is at least one of a correlation coefficient, weighted correlation coefficient, Euclidian distance or absolute maximum distance.

3. The method of claim 1, further comprising comparing said extracted spectrum (E) to a residual spectrum.

4. The method of claim 1, wherein said method is conducted in an iterative fashion.

5. The method of claim 1, wherein said regression includes at least one of

   a baseline spectrum,
   derivative spectra of spectra that are included in the regression,
   derivative spectra of spectra that are included in the regression, which are multiplied by an envelope function,
   and other component spectra to account for measurement errors or other variance in the spectral data.

6. The method of claim 1, further comprising using one or more regression coefficients to calculate concentrations.

7. The method of claim 1, wherein said regression includes at least one of multiple spectra (T) of a target component, multiple spectra (K') of a known component, a subset of the factor loadings of multiple spectra (T) of a target component, and a subset of the factor loadings of multiple spectra (K') of a known component.

8. The method of claim 1, wherein a plurality of spectral regions is used.

9. The method of claim 1, wherein;
   two or more regressions are performed for different target components, and different spectral regions are used for each different target component.

10. The method of claim 1, further comprising

    - grouping a number of target spectra (T) for each of a number of target components into clusters;
    - performing said regression for the target spectra (T) in each cluster, using either the mean of the target spectra (T), all of the target spectra (T), or a subset of the factors of the target spectra (T) in each cluster;
    - calculating the extracted spectrum (E) for each cluster; and
    - comparing the extracted spectra (E) to the clusters of target spectra (T) to determine which, if any, cluster the extracted spectrum (E) belongs to.

**11.** The method of claim 10, wherein either multiple spectra (K') of a known component are included in the regressions, or a subset of factor loadings of multiple spectra (K') of a known component is included in the regressions.

**12.** The method of claim 10, wherein the regressions include at least one of

a baseline spectrum,
derivative spectra of spectra that are included in the regression,
derivative spectra of spectra that are included in the regression, which are multiplied by an envelope function,
and other component spectra to account for measurement errors or other variance in the spectral data.

**13.** The method of claim 1, further comprising

- factoring a number of target spectra (T) for each of a number of target components
- grouping the factors into clusters;
- projecting the spectra to be analyzed and the spectra of the known components into the factor space;
- performing said regression for the target spectra (T) in each cluster in the factor space, using either the mean of the factor loadings of the target spectra (T), or all of the factor loadings of the target spectra (T);
- calculating the extracted spectrum (E) for each cluster; and
- either comparing the extracted spectra (E) and the target spectra (T) in the factor space, or projecting the extracted spectra (E) back to the original spectrum space and performing the comparison between the extracted spectra (E) and the target spectra (T) in the original spectrum space, to determine which, if any, cluster the extracted spectrum (E) belongs to.

**14.** The method of claim 13, wherein multiple spectra (K') of a known component are included in the regression.

**15.** The method in claim 13, wherein
the regressions include at least one of

a baseline spectrum,
derivative spectra of spectra that are included in the regression,
derivative spectra of spectra that are included in the regression, which are multiplied by an envelope function,
and other component spectra to account for measurement errors or other variance in the spectral data.

**Patentansprüche**

**1.** Computerimplementiertes Verfahren zum Bestimmen des Vorhandenseins einer spektralen Zielkomponente in einem Probenspektrum (S) eines Gemischs von Komponenten, wobei das Verfahren umfasst:

Durchführen einer Regression, die ein Zielspektrum (T) und die Spektren (K') einer oder mehrerer bekannter anderer Komponenten umfasst, die in dem Probenspektrum (S) des Gemischs vorhanden sind, wobei das Zielspektrum (T) das Spektrum der Zielkomponente ist, derart dass
$S = T * c_0 + K' * c' + R$, wobei $c_0$ ein skalarer Koeffizient ist, c' eine Matrix von Koeffizienten ist und R ein Rest ist
Berechnen eines gewonnenen Spektrums $E = S - K' * c'$,
wobei das gewonnene Spektrum (E) der Rest in dem Fall ist, in dem co auf Null gesetzt wird; und
Vergleichen des gewonnenen Spektrums (E) mit dem Zielspektrum (T) unter Verwendung einer Vergleichsmetrik zum Bestimmen des Vorhandenseins der Zielkomponente.

**2.** Verfahren nach Anspruch 1, wobei die Vergleichsmetrik mindestens eines von einem Korrelationskoeffizienten, gewichteten Korrelationskoeffizienten, euklidischen Abstand und/oder absoluten Höchstabstand ist.

**3.** Verfahren nach Anspruch 1, das ferner das Vergleichen des gewonnenen Spektrums (E) mit einem Restspektrum umfasst.

**4.** Verfahren nach Anspruch 1, wobei das Verfahren auf eine iterative Weise durchgeführt wird.

**5.** Verfahren nach Anspruch 1, wobei die Regression mindestens eines von Folgendem umfasst:

ein Basisspektrum,
Ableitungsspektren von Spektren, die in der Regression umfasst sind,
Ableitungsspektren von Spektren, die in der Regression umfasst sind, die mit einer Hüllfunktion multipliziert werden,
und andere Komponentenspektren zum Berücksichtigen von Messfehlern oder anderen Schwankungen in den Spektraldaten.

6. Verfahren nach Anspruch 1, das ferner das Verwenden eines oder mehrerer Regressionskoeffizienten zum Berechnen von Konzentrationen umfasst.

7. Verfahren nach Anspruch 1, wobei die Regression mindestens eines von mehreren Spektren (T) einer Zielkomponente, mehreren Spektren (K') einer bekannten Komponente, einer Untermenge der Faktorladungen mehrerer Spektren (T) einer Zielkomponente und/oder einer Untermenge der Faktorladungen mehrerer Spektren (K') einer bekannten Komponente umfasst.

8. Verfahren nach Anspruch 1, wobei eine Vielzahl von Spektralregionen verwendet wird.

9. Verfahren nach Anspruch 1, wobei:
zwei oder mehr Regressionen für verschiedene Zielkomponenten durchgeführt werden und verschiedene Spektralregionen für jede verschiedene Zielkomponente verwendet werden.

10. Verfahren nach Anspruch 1, ferner umfassend:

- Gruppieren einer Anzahl von Zielspektren (T) für jede einer Anzahl von Zielkomponenten in Cluster;
- Durchführen der Regression für das Zielspektrum (T) in jedem Cluster unter Verwendung entweder des Mittels der Zielspektren (T), sämtlicher Zielspektren (T) oder einer Untermenge der Faktoren der Zielspektren (T) in jedem Cluster;
- Berechnen des gewonnenen Spektrums (E) für jeden Cluster; und
- Vergleichen der gewonnenen Spektren (E) mit den Clustern von Zielspektren (T) zum Bestimmen, zu welchem Cluster das gewonnene Spektrum (E) gegebenenfalls gehört.

11. Verfahren nach Anspruch 10, wobei entweder mehrere Spektren (K') einer bekannten Komponente in den Regressionen umfasst sind oder eine Untermenge von Faktorladungen mehrerer Spektren (K') einer bekannten Komponente in den Regressionen umfasst ist.

12. Verfahren nach Anspruch 10, wobei die Regressionen mindestens eines von Folgendem umfassen:

ein Basisspektrum,
Ableitungsspektren von Spektren, die in der Regression umfasst sind,
Ableitungsspektren von Spektren, die in der Regression umfasst sind, die mit einer Hüllfunktion multipliziert werden,
und andere Komponentenspektren zum Berücksichtigen von Messfehlern oder anderen Schwankungen in den Spektraldaten.

13. Verfahren nach Anspruch 1, ferner umfassend:

- Faktorisieren einer Anzahl von Zielspektren (T) für jede von einer Anzahl von Zielkomponenten
- Gruppieren der Faktoren in Cluster;
- Projizieren der zu analysierenden Spektren und der Spektren der bekannten Komponenten in den Faktorraum;
- Durchführen der Regression für die Zielspektren (T) in jedem Cluster in dem Faktorraum unter Verwendung entweder des Mittels der Faktorladungen der Zielspektren (T) oder sämtlicher Faktorladungen der Zielspektren (T);
- Berechnen des gewonnenen Spektrums (E) für jeden Cluster; und
- entweder Vergleichen der gewonnenen Spektren (E) und der Zielspektren (T) in dem Faktorraum oder Projizieren der gewonnenen Spektren (E) zurück in den ursprünglichen Spektralraum und Ausführen des Vergleichs zwischen den gewonnenen Spektren (E) und den Zielspektren (T) im ursprünglichen Spektralraum, zum Bestimmen, zu welchem Cluster das gewonnene Spektrum (E) gegebenenfalls gehört.

**14.** Verfahren nach Anspruch 13, wobei mehrere Spektren (K') einer bekannten Komponente in der Regression umfasst sind.

**15.** Verfahren nach Anspruch 13, wobei:
die Regressionen mindestens eines von Folgendem umfassen:

ein Basisspektrum,
Ableitungsspektren von Spektren, die in der Regression umfasst sind,
Ableitungsspektren von Spektren, die in der Regression umfasst sind, die mit einer Hüllfunktion multipliziert werden,
und andere Komponentenspektren zum Berücksichtigen von Messfehlern oder anderen Schwankungen in den Spektraldaten.

## Revendications

**1.** Procédé mis en œuvre par ordinateur pour la détection de la présence d'une composante cible spectrale dans un spectre d'échantillon (S) d'un mélange de composantes, ledit procédé consistant à :

réaliser une régression qui comprend un spectre cible (T) et le spectre (K') d'une ou plusieurs autres composantes connues, présentes dans le spectre d'échantillon (S) du mélange, le spectre cible (T) étant le spectre de la composante cible, de sorte que $S = T * c_0 + K' * c' + R$, où $c_0$ est un coefficient scalaire, c'est une matrice de coefficients, et R est un résidu
calculer un spectre extrait $E = S - K'*c'$,
où ledit spectre extrait (E) est le résidu dans le cas où co est réglé à zéro ; et
comparer ledit spectre extrait (E) audit spectre cible (T) en utilisant un paramètre de comparaison pour détecter la présence de la composante cible.

**2.** Procédé selon la revendication 1, dans lequel ledit paramètre de comparaison est au moins un parmi un coefficient de corrélation, un coefficient de corrélation pondéré, une distance euclidienne ou une distance maximale absolue.

**3.** Procédé selon la revendication 1, consistant en outre à comparer ledit spectre extrait (E) à un spectre de résidu.

**4.** Procédé selon la revendication 1, dans lequel ledit procédé est réalisé d'une manière itérative.

**5.** Procédé selon la revendication 1, dans lequel ladite régression comprend au moins un élément parmi
un spectre de base,
des spectres de dérivation de spectres qui sont compris dans la régression,
des spectres de dérivation de spectres qui sont compris dans la régression, lesquels sont multipliés par une fonction d'enveloppe,
et d'autres spectres de composantes pour tenir compte des erreurs de mesure ou d'autres variances dans les données spectrales.

**6.** Procédé selon la revendication 1, consistant en outre à utiliser un ou plusieurs coefficients de régression pour calculer des concentrations.

**7.** Procédé selon la revendication 1, dans lequel ladite régression comprend au moins un parmi une pluralité de spectres (T) d'une composante cible, une pluralité de spectres (K') d'une composante connue, un sous-ensemble de saturations factorielles d'une pluralité de spectres (K') d'une composante cible et un sous-ensemble de saturations factorielles d'une pluralité de spectres (K') d'une composante connue.

**8.** Procédé selon la revendication 1, dans lequel une pluralité de régions spectrales sont utilisées.

**9.** Procédé selon la revendication 1, dans lequel :
deux régressions ou plus sont réalisées pour différentes composantes cibles et différentes régions spectrales sont utilisées pour chaque composante cible différente.

**10.** Procédé selon la revendication 1, comprenant en outre

- le regroupement d'un certain nombre de spectres cibles (T) pour chacune d'un certain nombre de composantes cibles en grappes ;
- la réalisation de ladite régression pour les spectres cibles (T) dans chaque grappe, en utilisant soit la moyenne des spectres cibles (T), tous les spectres cibles (T) ou un sous-ensemble de facteurs des spectres cibles (T) dans chaque grappe ;

le calcul du spectre extrait (E) pour chaque grappe ; et
la comparaison des spectres extraits (E) aux grappes de spectres cibles (T) pour déterminer à quelle grappe, s'il y en a une, le spectre extrait (E) appartient.

11. Procédé selon la revendication 10, dans lequel soit une pluralité de spectres (K') d'une composante connue sont compris dans les régressions ou un sous-ensemble de saturations factorielles d'une pluralité de spectres (K') d'une composante connue est compris dans les régressions.

12. Procédé selon la revendication 10, dans lequel les régressions comprennent au moins un élément parmi
un spectre de base,
des spectres de dérivation de spectres qui sont compris dans la régression,
des spectres de dérivation de spectres qui sont compris dans la régression, lesquels sont multipliés par une fonction d'enveloppe,
et d'autres spectres de composantes pour tenir compte des erreurs de mesure ou d'autres variances dans les données spectrales.

13. Procédé selon la revendication 1, comprenant en outre

- la factorisation d'un certain nombre de spectres cibles (T) pour chacune d'un certain nombre de composantes cibles
- le regroupement des facteurs en grappes ;
- l'envoi des spectres à analyser et des spectres des composantes connues dans l'espace factoriel ;
- la réalisation de ladite régression pour les spectres cibles (T) dans chaque grappe dans l'espace factoriel, en utilisant soit la moyenne des saturations factorielles des spectres cibles (T) ou toutes les saturations factorielles des spectres cibles (T) ;
- le calcul du spectre extrait (E) pour chaque grappe ; et
- soit la comparaison des spectres extraits (E) et des spectres cibles (T) dans l'espace factoriel, ou le renvoi des spectres extraits (E) à l'espace de spectre original et effectuer la comparaison entre les spectres extraits (E) et les spectres cibles (T) dans l'espace de spectre original, pour déterminer à quelle grappe, s'il y en a une, le spectre extrait (E) appartient.

14. Procédé selon la revendication 13, dans lequel une pluralité de spectres (K') d'une composante connue sont compris dans la régression.

15. Procédé selon la revendication 13, dans lequel
les régressions comprennent au moins un élément parmi
un spectre de base,
des spectres de dérivation de spectres qui sont compris dans la régression,
des spectres de dérivation de spectres qui sont compris dans la régression, lesquels sont multipliés par une fonction d'enveloppe,
et d'autres spectres de composantes pour tenir compte des erreurs de mesure ou d'autres variances dans les données spectrales.

Figure 1

Figure 2

Figure 3

Figure 4
Prior Art

Figure 5A

Figure 5B

Figure 5C

Figure 6

Figure 7A

Figure 7B

Figure 7C

Figure 8

Figure 9

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2366991 A1
- US 2009210194 A1
- EP 0535700 A2
- US 2011153226 A1

- US 5308982 A, Ivaldi
- US 7698098 B, Ritter
- US 20110213746 A, Botonjic-Sehic

### Non-patent literature cited in the description

- *Appl. Spec.,* 2012, vol. 66, 334

- Self-Weighted Correlation Coefficients and Their Application to Measure Spectral Similarity. *Appl. Spec.,* 2009, vol. 63, 916-919